# EUROPÄISCHE PATENTANMELDUNG

(11) **EP 0 854 141 A1**
(43) Veröffentlichungstag der Anmeldung: **22.07.1998**
(21) Anmeldenummer: 97121165.1
(22) Anmeldetag: 03.12.1997
(51) Int. Cl.: C07D 229/00

(54) **Verfahren zur Herstellung eines isocyanatfreien Uretdions des Isophorondiisocyanats**

(30) Priorität: 20.01.1997 DE 19701714
(71) Anmelder: HÜLS AKTIENGESELLSCHAFT, 45764 Marl (DE)
(72) Erfinder: Wolf, Elmar, Dr., 45661 Recklinghausen (DE)

(57) **Zusammenfassung**

Verfahren zur Herstellung eines isocyanuratfreien Uretdions des Isophorondiisocyanats, dadurch gekennzeichnet, daß man ein Isophorondiisocyanat mit einem Reinheitsgehalt von ≥ 99,9 Gew.-%, wobei die Konzentration der unbekannten Vorlaufkomponente im Gaschromatogramm des zur Dimerisierung eingesetzten Isophorondiisocyanats nicht größer als 0,05 Flachen-% beträgt, in einem inerten organischen Lösemittel oder ohne Lösemittel mit Hilfe von Katalysatoren der allgemeinen Formel: 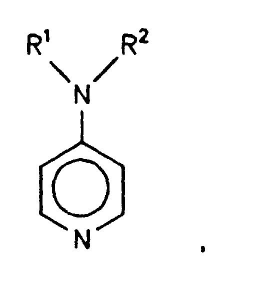 wobei R¹ und R² gleiche oder verschiedene Alkylreste mit 1 - 8 C-Atomen darstellen oder mit dem am Ring gebundenen N-Atom einen gemeinsamen 5- oder 6-Ring bilden können, der anstelle einer CH₂-Gruppe eine CH-CH₃-Gruppe, eine N-CH₃-Gruppe oder ein O-Atom enthalten kann, bei Temperaturen von 0 - 80 °C, dimerisiert und das gebildete Uretdion nach einem Umsatz von 5 - 70 % ohne vorhergehende Desaktivierung des Katalysators aus dem Reaktionsgemisch durch Dünnschichtdestillation bei 100 - 180 °C und 0,01 - 0,5 mbar isoliert.

## Beschreibung

Die vorliegende Erfindung betrifft ein Verfahren zur Herstellung eines Uretdions des 3-Isocyanatomethyl-3.5.5-trimethylcyclohexylisocyanats (Isophorondiisocyanat, IPDI).

Das Dimere des Isophorondiisocyanats (im folgenden IPDI-Uretdion abgekürzt) stellt eine wichtige Ausgangsverbindung zur Herstellung von blockierungsmittelfreien PUR-Pulverhärtern (DE 30 30 588, 30 30 539, 30 30 572) dar, die im Zuge der immer strenger werdenden staatlichen Umweltschutzauflagen zusehends an Bedeutung gewinnen.

(Cyclo)aliphatische Uretdione, u. a. auch IPDI-Uretdion, wurden erstmals in der DE-OS 16 70 720 beschrieben, wobei als Katalysatoren tertiäre Phosphine eingesetzt werden. Diese Uretdione enthalten jedoch in beträchtlichem Maße die entsprechenden Isocyanurate als Verunreinigung. Auch bei der Dimerisierung des IPDI entsprechend dem in der DE-OS 16 70 720 beschriebenen Verfahren wird kein Uretdion, sondern nur ein Gemisch von Reaktionsprodukten erhalten, das maximal aus 80 Gew.-% IPDI-Uretdion besteht. Der Rest ist nicht abtrennbares trimeres Isophorondiisocyanat.

Die DE-OS 19 34 763 befaßt sich ausschließlich mit der Oligomerisierung von IPDI mit tertiären Phosphinen.

Die gemäß der lehre dieser DE-OS erhaltenen Reaktionsprodukte bestehen aus ca. 60 Gew.-Teilen dimerem (in der Hitze spaltbarem) und trimerem bzw. höheroligomerisiertem (in der Hitze nicht mehr spaltbarem) IPDI. Durch geeignete Verfahrensvarianten (z. B. niedriger Umsatz) kann der Dimer-Anteil noch auf ca. 75 Gew.-Teile erhöht werden. Eine weitere Erhöhung des Uretdion-Gehaltes ist nicht mehr möglich, da der Katalysator (tert. Phosphine) nicht nur die Dimerisierung, sondern auch die Trimerisierung des IPDI zum entsprechenden Isocyanurat katalysiert.

In der DE-PS 30 30 513 wird zum ersten Mal ein isocyanuratfreies IPDI-Uretdion beschrieben. Als Katalysatoren werden Phosphorigsäuretriamide, speziell Trisdimethylaminophosphin, eingesetzt. Nachteilig bei diesem Verfahren ist, daß sich bei der kontinuierlichen Herstellung des IPDI-Uretdions, bei der der Katalysator im Kreis gefahren wird, Hexamethylphosphorsäuretriamid, das bekanntlich im Verdacht steht, cancerogen zu sein (Br. J. Cancer 38, 418 - 427 (1978)), im Reaktionsprodukt anreichert. Dieser Nachteil der Anreichetung eines cancerogen verdächtigen Stoffes im IPDI-Uretdion konnte durch Einsatz der in der DE-OS 37 39 549 beschriebenen Dimerisierungskatalysatoren für IPDI (in 4-Stellung substituierte Dialkylaminopyridine) beseitigt werden. Diese 4-Dialkylaminopyridine, speziell 4-Dimethylaminopyridin (DMAP), haben sich zur Herstellung von IPDI-Uretdion im technischen Maßstab durchgesetzt.

Bisher wurde zur Dimerisierung von IPDI eine IPDI-Qualität eingesetzt, wie sie durch Phosgenierung von Isophorondiamin erhalten wird (DE-PS 12 02 785):

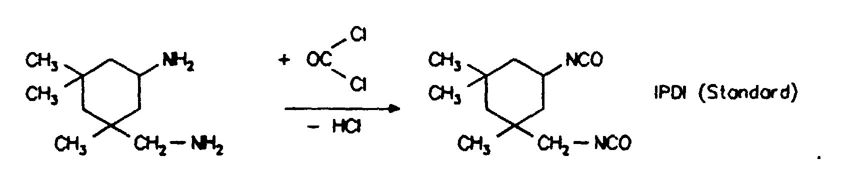

Dieses Standard-IPDI enthält ca. 180 mg/kg Gesamt-Chlor, davon beträgt der hydrolysierbare Chlorgehalt ca. 120 mg/kg.

In den DE-OSS 38 28 033 und 42 14 236 bzw. 42 31 417 wird ein Verfahren zur Herstellung von IPDI beschrieben, das auf den Einsatz des hochgiftigen Phosgens verzichtet:

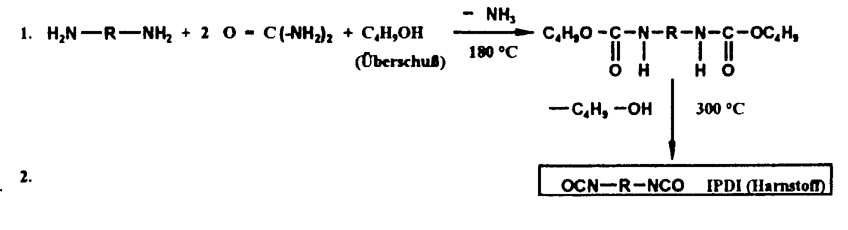

Bei diesem Verfahren fällt ein IPDI an, das chlorfrei ist und im folgenden mit IPDI (Harnstoff) abgekürzt werden soll. Vergleicht man die beiden IPDI-Qualitäten - IPDI (Standard) und IPDI (Harnstoff) (99,6 % IPDI (GC)) - hinsichtlich der Reaktion mit Alkoholen oder der Trimerisierung - so sind bei diesen Umsetzungen kaum Unterschiede festzustellen. Versucht man dagegen, das IPDI (Harnstoff) mit 4-Dimethylaminopyridin zu dimerisieren (DE-OS 37 39 549), so erhält man stark gefärbte Reaktionsprodukte, die obendrein sogar noch geringe Mengen an trimerem IPDI enthalten. Dieses Phänomen ist nicht zu erklären, da das Reaktionsverhalten des IPDI vom Verfahren seiner Herstellung unabhängig sein sollte. Ein IPDI, das zur Herstellung von IPDI-Uretdion nicht geeignet ist, wäre in seinen Einsatzmöglichkeiten stark eingeschränkt.

Aufgabe der vorliegenden Erfindung war es deshalb, ein nicht verfärbtes IPDI-Uretdion aus dem IPDI (Harnstoff) herzustellen.

Überraschenderweise konnte diese Aufgabe dadurch gelöst werden, daß zur Dimerisierung ein IPDI (Harnstoff) eingesetzt wurde, bei dem die Konzentration der unbekannten Vorlaufkomponenten höchstens 0,05 % (Flächen-% im GC) und die IPDI-Konzentration ≥ 99,9 Gew.-% ist.

Gegenstand der vorliegenden Erfindung ist ein Verfahren zur Herstellung eines isocyanuratfreien Uretdions des Isophorondiisocyanats, dadurch gekennzeichnet, daß man ein Isophorondiisocyanat mit einem Reinheitsgehalt von ≥ 99,9 Gew.-%, wobei die Konzentration der unbekannten Vorlaufkomponente im Gaschromatogramm des zur Dimerisierung eingesetzten Isophorondiisocyanats nicht größer als 0,05 Flächen-% beträgt, in einem inerten organischen Lösemittel oder ohne Lösemittel mit Hilfe von Katalysatoren der allgemeinen Formel: 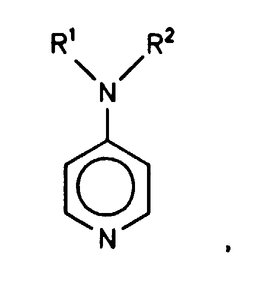 wobei R¹ und R² gleiche oder verschiedene Alkylreste mit 1 - 8 C-Atomen darstellen oder mit dem am Ring gebundenen N-Atom einen gemeinsamen 5- oder 6-Ring bilden können, der anstelle einer CH₂-Gruppe eine CH-CH₃-Gruppe, eine N-CH₃-Gruppe oder ein O-Atom enthalten kann, bei Temperaturen von 0 - 80 °C, dimerisiert und das gebildete Uretdion nach einem Umsatz von 5 - 70 % ohne vorhergehende Desaktivierung des Katalysators aus dem Reaktionsgemisch durch Dünnschichtdestillation bei 100 - 180 °C und 0,01 - 0,5 mbar isoliert.

Das zur Dimerisierung einsetzbare IPDI wird z. B. in EP 0568 782 beschrieben. Während sich bei der Umsetzung mit Polyolen und bei der Trimerisierung ein Gehalt an unbekannten Vorlaufkomponenten von 0,3 % (Flachen-% im GC) nicht nachteilig bemerkbar macht, dürfen diese unbekannten Vorlaufkomponenten bei einem zur Dimerisierung eingesetzten IPDI nicht größer als 0,05 % (Flächen-% im GC) beträgt, wenn starke Verfärbungen im Reaktionsprodukt vermieden werden sollen. Eine solche IPDI-Qualität erhält man in der Regel dann, wenn das entsprechend der EP 0568 782 hergestellte IPDI so fraktioniert wird, daß der Gehalt des IPDI ≥ 99,9 % beträgt. Bei den Dimerisierungskatalysatoren handelt es sich um 4-Dialkylaminopyridine, wie z. B. 4-Dimethylaminopyridin, 4-Diethylaminopyridin, 4-Pyrrolidinopyridin, 4-Piperidinopyridin und 4-(4-Methylpiperidino)-pyridin. Sie werden in Mengen von 0,2 - 4 Gew.-%, vorzugsweise 0,5 - 2 Gew.-%, eingesetzt. Besonders geeignet sind 4-Dimethylaminopyridin (DMAP) und 4-Pyrrolidinopyridin.

Das erfindungsgemäße Verfahren kann prinzipiell kontinuierlich und diskontinuierlich durchgeführt werden.

Die Durchführung des erfindungsgemäßen Verfahrens erfolgt bevorzugt derartig, daß zunächst IPDI (Harnstoff) mit Hilfe der beschriebenen Katalysatoren bis zu einem Umsatz, der die Förderung des Reaktionsgemisches im flüssigen Zustand bei Raumtemperatur noch zuläßt (ca. 40 - 60 % IPDI-Umsatz), umgesetzt wird, und danach das nicht umgesetzte IPDI (Harnstoff) mit dem Katalysator durch Dünnschichtdestillation, insbesondere durch Kurzwegdestillation, vom Reaktionsprodukt abgetrennt wird. Das abdestillierte IPDI (Harnstoff) plus Katalysator kann wieder zur Reaktion eingesetzt werden. Die Reaktionszeit, die Zeit, in der 40 - 60 % des IPDI (Harnstoff) umgesetzt sind, hängt (bei konstanter Temperatur) in hohem Maße von der Konzentration sowie von der Art des eingesetzten Katalysators ab. Sie beträgt in der Regel 40 - 90 h. Die Reaktion kann in polaren Lösungsmitteln, wie Estern, Ethern und Ketonen, oder lösungsmittelfrei durchgeführt werden. Bevorzugt arbeitet man lösungsmittelfrei. Die Aufarbeitung des Reaktionsgemisches erfolgt, wie bereits angeführt, durch Dünnschichtdestillation bei 100 - 180 °C und 0,01 - 0,5 mbar. Das so nach dem erfindungsgemäßen Verfahren hergestellte IPDI-Uretdion ist natürlich auch zur Herstellung von lösungsmittelfreien Ein- und Zweikomponenten-Lacken sowie Polyurethan-Pulverlacken geeignet.

### Experimental Teil

**A.Zur Dimerisierung eingesetzte IPDI-Qualitäten:** 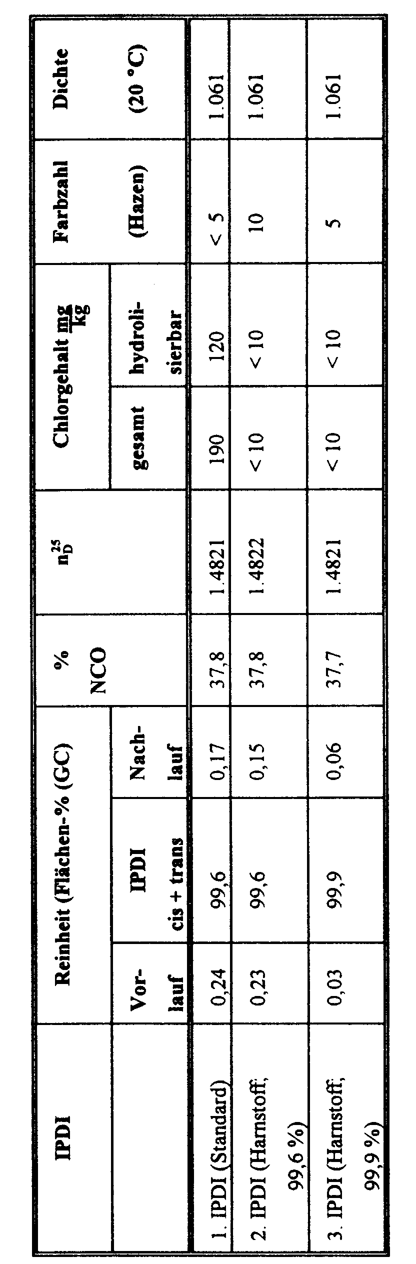
B. Die IPDI-Qualitäten 1 - 3 wurden mit 1 % DMAP versetzt und 3 Tage bei Raumtemperatur unter N₂-Abdeckung stehen gelassen. Der Reaktionsverlauf wurde durch Bestimmung des Brechungsindex und des NCO-Gehalts verfolgt.
   Nach einer Reaktionszeit von 3 d wurde das nicht umgesetzte IPDI durch Dünnschichtdestillation bei 120 °C/0,1 mbar vom Reaktionsprodukt abgetrennt. In der nachfolgenden Tabelle findet sich die Zusammensetzung der Reaktionsprodukte.

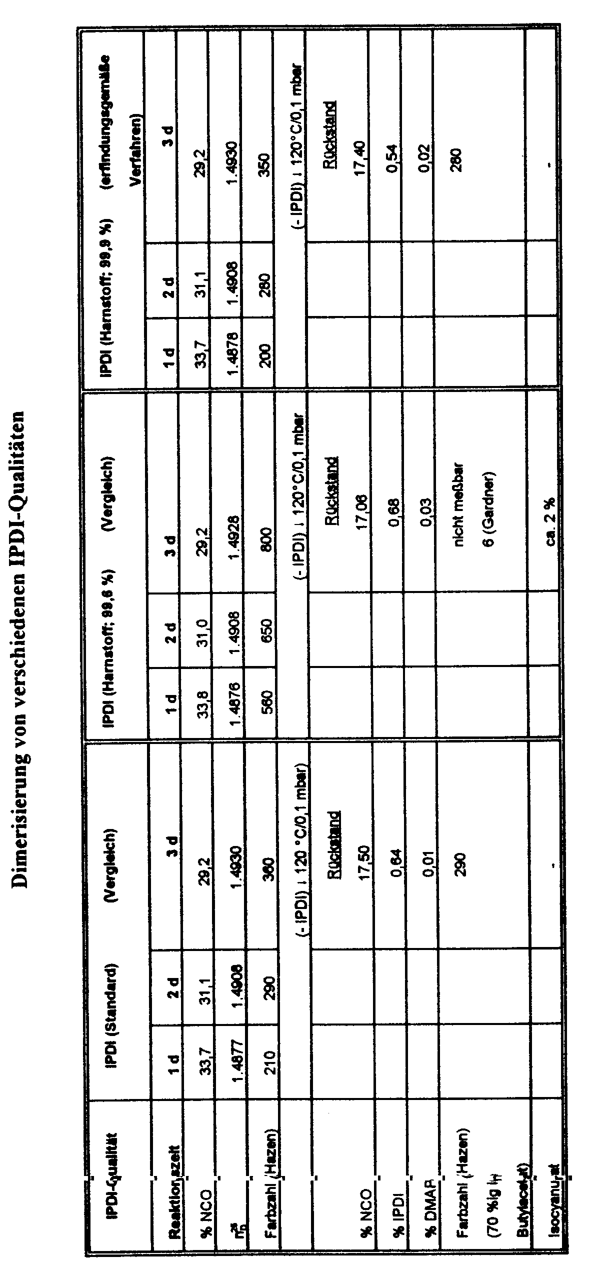

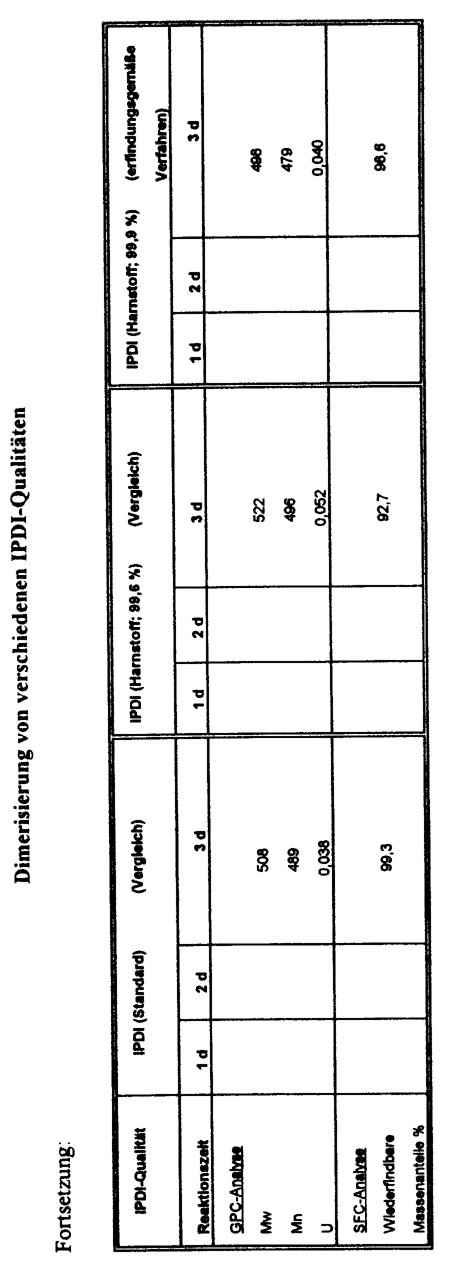

## Patentansprüche

1. Verfahren zur Herstellung eines isocyanuratfreien Uretdions des Isophorondiisocyanats,
dadurch gekennzeichnet,
daß man ein Isophorondiisocyanat mit einem Reinheitsgehalt von ≥ 99,9 Gew.-%, wobei die Konzentration der unbekannten Vorlaufkomponente im Gaschromatogramm des zur Dimerisierung eingesetzten Isophorondiisocyanats nicht größer als 0,05 Flächen-% beträgt, in einem inerten organischen Lösemittel oder ohne Lösemittel mit Hilfe von Katalysatoren der allgemeinen Formel: 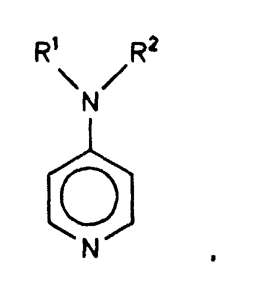 wobei R¹ und R² gleiche oder verschiedene Alkylreste mit 1 - 8 C-Atomen darstellen oder mit dem am Ring gebundenen N-Atom einen gemeinsamen 5- oder 6-Ring bilden können, der anstelle einer CH₂-Gruppe eine CH-CH₃-Gruppe, eine N-CH₃-Gruppe oder ein O-Atom enthalten kann, bei Temperaturen von 0 - 80 °C, dimerisiert und das gebildete Uretdion nach einem Umsatz von 5 - 70 % ohne vorhergehende Desaktivierung des Katalysators aus dem Reaktionsgemisch durch Dünnschichtdestillation bei 100 - 180 °C und 0,01 - 0,5 mbar isolierte

2. Verfahren nach Anspruch 1,
dadurch gekennzeichnet,
daß bei Temperaturen von 10 - 30 °C dimerisiert wird.

3. Verfahren nach den Ansprüchen 1 bis 2,
dadurch gekennzeichnet,
daß nach einem Umsatz von 20 bis 60 % isoliert wird.

4. Verfahren nach den Ansprüchen 1 bis 2,
dadurch gekennzeichnet,
daß nach einem Umsatz von 40 bis 50 % isoliert wird.

5. Verfahren nach den Ansprüchen 1 bis 4,
dadurch gekennzeichnet,
daß Katalysatoren ausgewählt aus der Gruppe 4-Dimethylaminopyridin, 4-Diethylaminopyridin, 4-Pyrrolidinopyridin, 4-Piperidinopyridin und 4-(4-Methylpiperidino)-pyridin, eingesetzt werden.

6. Verfahren nach den Ansprüchen 1 bis 5,
dadurch gekennzeichnet,
daß die Katalysatoren in Mengen von 0,2 bis 4 Gew.-% eingesetzt werden.

7. Verwendung eines Isophorondiisocyanats mit einem Reinheitsgehalt von ≥ 99,9 Gew.-%, wobei die Konzentration der unbekannten Vorlaufkomponente im Gaschromatogramm des zur Dimerisierung eingesetzten Isophorondiisocyanats nicht größer als 0,05 Flächen-% beträgt, zur Herstellung eines isocyanuratfreien Uretdions des Isophorondiisocyanats.

8. Isocyanuratfreies Uretdion des Isophorondiamins,
dadurch erhältlich,
daß man ein Isophorondiisocyanat mit einem Reinheitsgehalt von ≥ 99,9 Gew.-%, wobei die Konzentration der unbekannten Vorlaufkomponente im Gaschromatogramm des zur Dimerisierung eingesetzten Isophorondiisocyanats nicht größer als 0,05 Flächen-% beträgt, in einem inerten organischen Lösemittel oder ohne Lösemittel mit Hilfe von Katalysatoren der allgemeinen Formel: 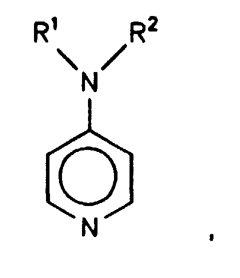 wobei R¹ und R² gleiche oder verschiedene Alkylreste mit 1 - 8 C-Atomen darstellen oder mit dem am Ring gebundenen N-Atom einen gemeinsamen 5- oder 6-Ring bilden können, der anstelle einer CH₂-Gruppe eine CH-CH₃-Grüppe, eine N-CH₃-Gruppe oder ein O-Atom enthalten kann, bei Temperaturen von 0 - 80 °C, dimerisiert und das gebildete Uretdion nach einem Umsatz von 5 - 70 % ohne vorhergehende Desaktivierung des Katalysators aus dem Reaktionsgemisch durch Dünnschichtdestillation bei 100 - 180 °C und 0,01 - 0,5 mbar isoliert.
